# EUROPEAN PATENT APPLICATION

(11) **EP 4 744 864 A1**
(43) Date of publication of application: **20.05.2026**
(21) Application number: 24213494.8
(22) Date of filing: 18.11.2024
(51) Int. Cl.: B29C 64/118, B33Y 10/00, B33Y 80/00, A61F 2/28

(54) **MANUFACTURING OBJECTS WITH SEAMLESS SOLID-TO-POROUS TRANSITIONS**

(71) Applicant: Kumovis GmbH, 81369 München (DE)
(72) Inventor: BRUYAS, Arnaud, 81549 München (DE); KRIEGER, Yannick, 81541 München (DE); LEONHARDT, Stefan, 81543 München (DE); PAMMER, Sebastian, 81677 München (DE)
(74) Representative: DTS Patent- und Rechtsanwälte PartmbB

(57) **Abstract**

A method (200) of fabricating an object, such as a three-dimensional object comprising variable-density regions (104), preferably using a fused filament fabrication technique, comprising the steps of: depositing (202) a material to create a first layer of the object based on a first pathway in the X, Y and Z dimensions, wherein the first layer (100) comprises first regions (102) including a first infill density, preferably porous regions; and depositing (204) the material to create second regions (104) by filling selected pores of the porous regions (102) within the first layer (100) based on a second pathway in the X, Y and Z dimensions, wherein the second regions (104) include a second infill density; wherein the second infill density is greater than the first infill density.

## Description

The present invention relates to a method of fabricating an object using a 3D printing technique. Specifically, the present invention relates to a method of fabricating an object, such as a three-dimensional object comprising variable density regions, preferably using a fused filament fabrication technique.

Fused Filament Fabrication (FFF) is a widely adopted additive manufacturing technology known for its simplicity, versatility, and accessibility. This process involves the extrusion of thermoplastic filament through a heated nozzle to create three-dimensional objects layer by layer, following a specific path to build up the geometry of a part. While FFF has been successfully utilized across various industries-including healthcare, aerospace, automotive, and consumer products-it presents significant challenges when printing components that require distinct volumetric properties, particularly those that incorporate both solid (high-density) and porous (low-density) structures.

Traditionally, when volumes or regions of different densities need to be printed within the same object, manufacturers apply sequential deposition methods. In this approach, porous regions are printed first in one layer, followed by the deposition of solid regions in a subsequent layer. To adjust the properties within different parts of a single object, manufacturers use varying infill patterns and densities. For instance, a solid region (area) might have 100% infill density, making it denser and more rigid, while a porous region might have a lower infill density, creating a lightweight, flexible structure. Typically, each layer is printed with a rectilinear infill, where the extruder follows a repeating path, laying down parallel lines in one direction and then alternating in the next layer (a grid-like pattern). This approach is effective for creating distinct volumes within an object, but it introduces notable limitations. The transition between solid and porous regions results in visible borders with different densities because the extruder has to "turn" at the edge of each region, creating turning point. These points cause the transition between solid and porous regions to be abrupt, leading to structural discontinuities and potential stress concentration areas.

One of the primary challenges arising from this traditional approach is the creation of pronounced turning points at the interface between solid and porous areas. These turning points result from abrupt changes in infill density and deposition strategies, leading to weak mechanical bonds and potential failure points in the final printed part. Such inconsistencies not only compromise the structural integrity of the component but also detract from the overall aesthetic quality of the finished product. Furthermore, the inability to effectively control the transition between different volumetric properties limits design possibilities and functionality.

As industries increasingly demand lightweight, high-performance parts with varying densities and structural characteristics, the limitations of existing FFF techniques become more pronounced. Therefore, there exists a pressing need for an improved method of FFF that enables the seamless integration of solid and porous structures within a single component, leveraging distinct isotropic and anisotropic fill strategies. Such advancements would enhance mechanical performance, optimize material usage, and broaden the scope of applications for FFF technology.

It is therefore an object of the present invention to address the issue of abrupt boundaries by improving the fabrication method for creating seamless transitions from porous regions to solid regions within a single object (a part or a component).

This object is achieved in the present invention by a method according to claim 1. In particular, a method of fabricating an object including variable density regions, such as a three-dimensional object comprising porous and solid regions, preferably using a fused filament fabrication technique, is provided. The method comprises the steps of:
- depositing (202) a material to create a first layer of the object based on a first pathway in the X, Y and Z dimensions, wherein the first layer (100) comprises first regions (102) including a first infill density, preferably porous regions;
- depositing (204) the material to create second regions (104) by filling selected pores of the porous regions (102) within the first layer (100) based on a second pathway in the X, Y and Z dimensions, wherein the second regions (104) include a second infill density;
   wherein the second infill density is greater than the first infill density.

The invention is based on the basic idea to fabricate an object including seamless solid to porous transitions using a 3D printing technique, preferably using the FFF technique. The object comprises solid and porous volumes or regions. Accordingly, initially, in step A, a porous structure (a porous region) is printed within the first layer. Next, in step B, a denser structure (a solid region or a high-density region) is printed within the same first layer by selectively filling some of the pores created in the first layer during step A while also selectively leaving other pores open. The printing steps A and B are therefore performed in the first layer without changing the Z dimension of a printer head. In step A, initial pathways are formed, and in step B, new pathways are intentionally crossed with those from step A. This overlap creates a region having a density higher than the density of the porous structure in the first layer that seamlessly connects to the porous structure. This ensures that the transitions between two regions of variable densities are smooth and free from abrupt density changes, which can compromise structural integrity. As a result, the finished object or part not only exhibits a seamless appearance but also benefits from increased durability and strength across the transition zones. Advantageously, the object fabricated using the method according to the present invention is suitable for applications where both flexibility in the porous areas and rigidity in the solid areas are essential.

In particular, the present invention seeks to print objects or parts that incorporate both dense and porous regions within the same layer, ensuring a seamless transition between these areas and achieving uniform mechanical properties throughout the entire object. Advantageously, the smooth transition between solid and porous regions enhances structural integrity by reducing stress concentrations at the transition zone (or area). This makes the object more resistant to mechanical stresses, as there are no abrupt changes in density that could act as weak points. Further, the seamless transition between densities results in a cleaner, more visually appealing object. Additionally, the printer's operation is enhanced, as the extruder no longer needs to make sharp turns at the boundaries between different regions.

In particular, at a first step, a porous layer is deposited. The deposition is carried out to form a first layer with uniformly distributed porous regions, such that no unprinted or undeveloped areas remain within the first layer. Thereafter, the solid regions are overlaid as necessary to enhance the density of the object in the selected regions within the first layer. The overlaying of the solid regions occurs at the same height.

In particular, the first pathway and the second pathway comprise overlapping segments that cross each other in the X and Y dimensions, wherein the first pathway and the second pathway further comprise a substantially consistent Z dimension, with a minimal vertical offset. The Z-height of the first and second pathways is maintained within a tolerance range for effectively preventing significant vertical offset between pathways.

In particular, the first regions comprise the first infill density varying between 0 to 30% infill.

In particular, the second regions comprise the second infill density varying between 30% and 40% infill, 40% and 60% infill, or 70% and 100% infill.

For example, the second regions comprise solid regions with 100% infill density.

In particular, the method further comprises repeating the steps of depositing to create a subsequent porous layer and to fill the selected pores within the first layer, and/or the step of adapting printing parameters for creating (depositing) the solid regions, wherein the printing parameters comprise at least one of extrusion temperature, print speed, and retraction settings.

This ensures advantageously proper deposition of the material and optimal layer adhesion during the printing process.

In particular, the material comprises a filament material selected from the group comprising high performance polymers, such as Polyaryletherketones (PAEKs), Polyetheretherketone (PEEK), Polyetherketoneketone (PEKK), Polyphenylsulfone (PPSU), and polymers filled with Biphasic Calcium Phosphate/Hydroxyapatite (BCP/HA), bio-degradable polymers, such as poly(L-lactic acid) (PLLA), Poly(D-lactic acid) (PDLA), Poly(lactic-co-glycolic acid) (PLGA), Poly(glycolic acid) (PGA), Poly(dioxanone) (PDO), and Polycaprolactone (PCL).

In particular, the material comprises a filament material selected from the group comprising polylactic acid (PLA), acrylonitrile butadiene styrene (ABS), and thermoplastic polyurethane (TPU).

In particular, the method further comprises the step of employing a temperature control system to monitor and adjust the nozzle temperature during extrusion.

In particular, the first pathway is configured to create a porous structure, e.g. including open spaces (pores). These pores can vary in shape, size, and arrangement depending on the specific pattern or structure chosen for the pathway.

In particular, the first layer comprises porous regions including interconnected pores.

In particular, the first pathway comprises Triply Periodic Minimal Surfaces including patterns such as Gyroid, Schwarz, Diamond.

Advantageously, these types of patterns allow for optimal mechanical properties and can be adapted for specific uses in medical, engineering, or structural applications.

In particular, the first pathway may also comprise a rectilinear refill pattern, a honeycomb pattern, a triangular pattern, a concentric pattern, a zigzag patent or a combination thereof.

In particular, the first pathway pattern can be optimized using FEA (Finite Element Analysis) to ensure that it has the desired mechanical properties. FEA can help determine the best design for strength, flexibility, and durability based on specific requirements.

In particular, the first pathway is configured to have anisotropic properties. Advantageously, the mechanical behavior (such as stiffness, strength, or elasticity) are tailored to perform differently in different directions. This is important in many applications, such as implants or load-bearing structures, where the material needs to behave differently in response to varying stresses or forces.

In particular, the second pathway is configured to create a region of significantly higher infill density (e.g. 70%, 80% or 100%) compared to the first pathway. For example, the first pathway comprises patterns, such as rectilinear (with parallel paths) or concentric.

The high-density regions (e.g. solid regions) are designed to achieve specific mechanical properties, allowing for the creation of parts with patient-matched or patient-specific mechanical properties. These patient-specific properties can be derived from biomechanical or FEA (Finite Element Analysis) simulations.
the method further comprises the step of preparing, which includes at least one of the following steps
- providing a thermoplastic filament material;
- feeding the filament material into a print head comprising an extrusion nozzle;
- heating a nozzle of a printer to a temperature sufficient to melt the filament;
- extruding the melted filament through the nozzle onto a build platform;

In particular, the method further comprises the step of utilizing a path tool to define predefined pathways for a print head movement along the X, Y, and Z dimensions; and the step of moving the print head along the defined pathways in the X, Y, and Z dimensions to form the object.

In particular, the method further comprises the step of cooling the extruded filament to solidify the layer preferably the cooling step utilizes a fan system to accelerate the solidification of the filament.

In particular, the method further comprises the step of post-processing the printed object to enhance surface finish and mechanical properties.

The present invention further relates to an object fabricated by the method as described above. In particular, the object comprises porous and solid regions with seamless transitions between the solid and porous regions.

It is shown in
- **Fig. 1**: a schematic view of a layer of an object comprising both a solid volume and a porous volume to be fabricated using a method according to the prior art and the present invention;
- **Fig. 2**: another schematic view of a layer of an object comprising both a solid and porous volume to be fabricated using a method according to the prior art and the present invention; and
- **Fig. 3**: a block diagram of a method for manufacturing an object according to the present invention.

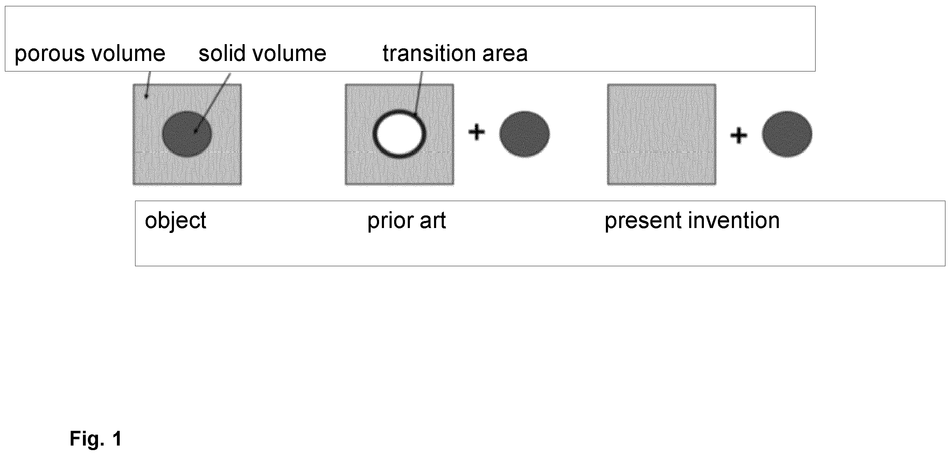

**Fig. 1** shows a schematic view of a layer of an object comprising both a solid volume and a porous volume.

In traditional FFF printing method according to the prior art, when different volumes need to be printed within the same object or part, the typical approach is to print these volumes sequentially. The traditional method further comprises adjusting both the infill density and pattern at specific "transition points" within the print layers.

For example, if a part has a solid core and a porous outer structure, the printer first deposits the porous volume using a lower infill density (less than 100%) and open patterns to introduce intentional gaps. Subsequently, for solid regions, the printer employs a dense, overlapping pattern-often lines or a grid with a high infill percentage-ensuring minimal gaps between extruded lines for a strong, compact structure.

The transition from porous to solid creates an abrupt change in density and composition, which results pronounced turning points at the transition. These turning points can negatively impact both the mechanical properties and aesthetic quality of the printed object. The transition zone may exhibit weaknesses or poorly defined characteristics, which can lead to performance issues, particularly in load-bearing applications. Specifically, the mechanical properties and appearance may suffer due to the poor characteristics at these turning points, which can result in performance issues, particularly in load-bearing applications.

In the FFF method according to the present invention, the turning points are optimized to create an object with improved strength and visual appeal.

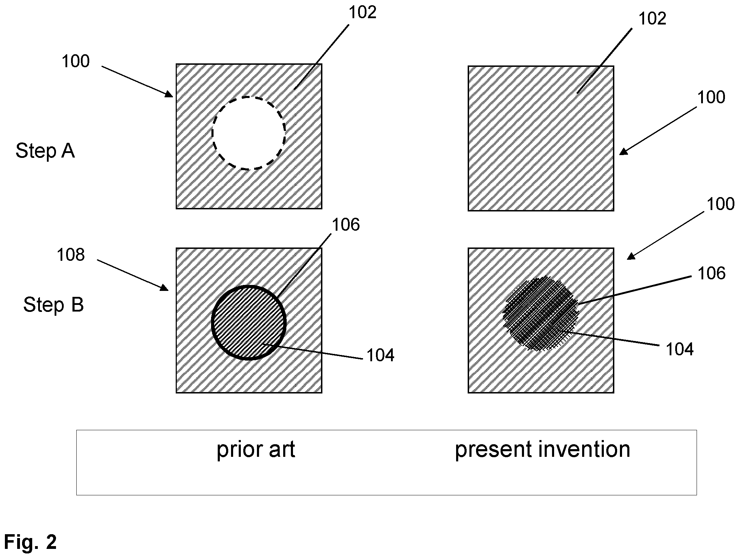

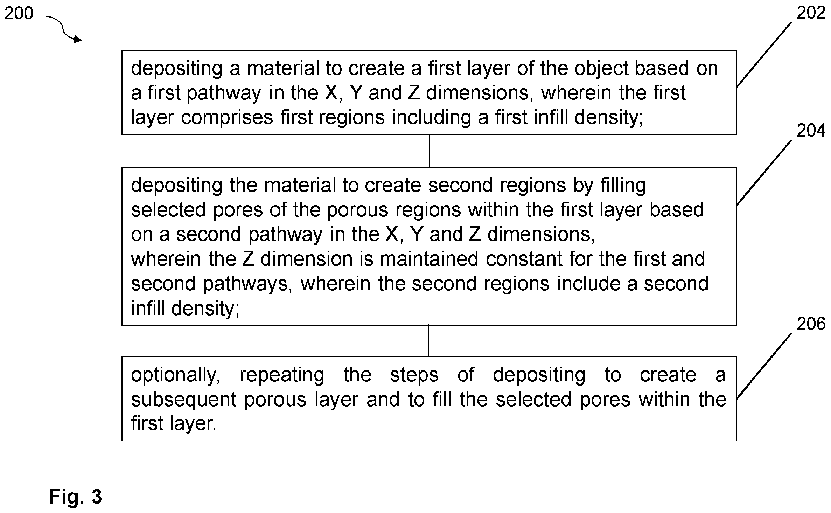

**Fig. 2** shows another schematic view of a layer 100 of an object that comprises both a solid volume (or region) 104 and a porous volume (region) 102, fabricated using a method according to the prior art and a method 200 (shown in Fig. 3) according to the present invention.

In the method according to the prior art, a printer begins by depositing a first layer 10 including a first region (porous region) 12 in accordance with a specific pattern, while leaving a second region 14 of a higher density (a solid region) unprinted (step A). The first layer 10 features a lower infill density and an open pattern.

Following this, at step B according to the prior art, the printer changes the Z position (direction perpendicular to the plane shown in view) of a printer head to deposit the solid region 14 in a subsequent layer 18 with a higher infill density. The solid region 18 has a dense pattern, often incorporating lines or grids with minimal gaps between an extruded filament. The transition between the porous and solid regions 12, 14 occurs abruptly, resulting in a clear transition boundary 16 (shown as a solid circle).

In the method 200 according to the present invention, at step A, a first layer 100 (i.e. the porous layer) is deposited. The deposition is performed to create a first layer 100 with uniform porous regions, ensuring that no unprinted or undeveloped areas remain in the first layer.

The pattern used for depositing the porous layer 100 is not restricted to rectilinear or parallel struts. The pattern can encompass a wide range of designs tailored to specific requirements. This flexibility allows for the incorporation of various geometric configurations, such as hexagonal grids, concentric circles, or more complex lattice structures, depending on the intended application and desired mechanical properties.

By varying the deposition pattern, the porosity, strength, and overall performance of the printed object can be optimized. This in turn enables customization that meets diverse functional and aesthetic needs.

For example, the first pathway may include various refill patterns such as rectilinear, honeycomb, triangular, concentric, or zigzag, or combinations thereof.

The first pathway can be optimized using FEA to achieve desired mechanical properties, ensuring strength, flexibility, and durability.

The first pathway is also configured to have anisotropic properties, allowing for different mechanical behaviors (e.g., stiffness, strength, elasticity) in different directions, which is crucial for applications like implants or load-bearing structures.

In particular, the second pathway creates a high-density region (e.g., 70%, 80%, or 100% infill density) to enhance mechanical properties. The high-density regions can, for example, derived from biomechanical or FEA simulations.

At step B, the solid region 104 is printed by filling preselected pores within the porous layer 100 according to a specific pattern. That is, the second regions 104 with higher density than the first regions 102 are deposited almost in the same the Z-position of the print head in the step A and the step B. Further, not all of the pores in the porous layer 100 are filled during the deposition in step B. This allows for maintaining a controlled level of porosity even after the solid material is deposited.

The pathway for depositing the second region 104 does not follow the pathway for depositing the first region 102. The pathway for depositing the second region 104 however crosses the pathway of the first region. By carefully managing the filling of the pores, it is possible to tailor the mechanical properties of the object, thereby achieving a balance between strength and weight that is optimized for specific applications.

The pathway for depositing the second region 104 does not replicate the pathway used for the first porous region 102. However, it is important to note that the solid deposition pathway intersects with the porous deposition pathway. This deliberate crossing allows for a seamless transition between the first and second regions 102, 104. In cases where different materials are used, this may result in the integration of the two different material properties, enabling the solid material to bond effectively with the porous layer without compromising the structural integrity.

**Fig. 3** shows a block diagram of a method 200 of fabricating an object according to the present invention.

The object comprises a three-dimensional object including first regions 102 and second regions 104 (porous volume and solid volume).

The method 200 comprises the step depositing 202 a filament material, using a fused filament fabrication technique, to create a first layer 100 of the object based on a first pathway in the X, Y and Z dimensions. The first layer 100 comprises porous regions that are uniformly distributed in the X and Y dimensions. This ensures that no unprinted regions is remained in the first layer 100.

The method 200 further comprises the step of depositing 204 the filament material to create solid regions by filling selected pores of the porous region of the first layer 100 based on a second pathway in the X, Y and Z dimensions.

During the steps of depositing 202, 204 the Z dimension of the first and second pathways is maintained at a constant value. That is, the height of the printer head remains unchanged.

The first pathway and the second pathway comprise overlapping segments that cross each other in the X and Y dimensions, in particular in the solid regions 104.

Advantageously, at a first step, a porous layer 100 is deposited. Thereafter, the second regions 104 having higher infill density than the first regions 102 are overlaid as necessary to enhance the density of the object in the selected first (porous) regions 102 within the first layer 100. In other words, the overlaying of the second regions 104 occurs at the same layer height. This results in a seamless transition from solid to porous regions within a single part.

The method 200 further comprises repeating 206 the steps of depositing 202, 204 to create a subsequent porous layer and to fill the selected pores within the first layer 100.

The method 200 further comprises the step of adapting printing parameters for creating (depositing) the solid regions.

The printing parameters comprise extrusion temperature, print speed, and/or retraction settings, each optimized to ensure a dense, cohesive deposition for solid areas.

For example, by adjusting the extrusion temperature, the material can flow smoothly and adhere effectively, which is essential for high-density regions. Similarly, print speed can be calibrated to balance precision and build time. Retraction settings help reduce unwanted drips of material that can occur when the nozzle moves over different parts of the object. Thus, gapes or weak spots can be prevented. This in turn leads to a uniform structure across solid and porous regions.

The filament material is selected from the group comprising polylactic acid (PLA), acrylonitrile butadiene styrene (ABS), and thermoplastic polyurethane (TPU).

Alternatively, the filament material high performance polymers (PAEKs, PEEK, PEKK, polymers filled with BCP/HA, PPSU, etc.) and bio-degradable polymers (PLLA, PDLA, PLGA, PGA, PDO, PCL, etc.).

In particular, the method further comprises the step of employing a temperature control system to monitor and adjust the nozzle temperature during extrusion.

The method 200 is not limited to a specific pattern and allows for flexibility in achieving the desired structure within each layer. For instance, the first pathway for the porous region and the second pathway for the solid region can independently employ patterns such as rectilinear, honeycomb, triangular, concentric, zigzag, or any combination of these.

This versatility enables precise control over the mechanical properties, as different patterns offer varying levels of strength, flexibility, and material usage. Additionally, using distinct patterns for the porous and solid regions can enhance the cohesion at the transition, ensuring a seamless integration of the regions within each layer.

The method 200, for example, begins with the preparation step, which involves providing a thermoplastic filament material suitable for extrusion.

Next, the filament is fed into the print head, which contains an extrusion nozzle.

The nozzle of the printer is then heated to a temperature sufficient to melt the filament, making it pliable for extrusion. This controlled heating allows the filament to reach its optimal viscosity for layering. This in turn, facilitates smooth deposition and strong interlayer adhesion.

In the extrusion step, the melted filament is pushed through the nozzle and deposited onto a build platform. This process forms the foundation of each layer, creating a stable base for subsequent layers and allowing for intricate shapes to be built up in successive steps.

In particular, a path tool is used to define the pathways for print head movement along the X, Y, and Z dimensions. By setting these predefined pathways, the method achieves accuracy and repeatability.

Finally, the print head moves along the defined pathways in the X, Y, and Z dimensions to form the object. This coordinated movement allows for the creation of both solid and porous regions within each layer, providing flexibility in designing the object's structure.

The method may further comprise the step of cooling the extruded filament to solidify the layer.

For example, the cooling step utilizes a fan system to accelerate the solidification of the filament.

The method 200 further comprises the step of post-processing the printed object to enhance surface finish and mechanical properties.

For example, the post-processing comprises at least one of a milling step, a grinding step, and a cutting step.

Present invention further relates to an object fabricated by the method 200.

The object comprises regions with variable infill densities, e.g. porous regions and solid regions, with seamless transitions between these regions. This ensures consistent structural integrity and eliminating abrupt boundaries that could weaken the part.

The ability to customize porosity and density in different sections of the object allows for improved material efficiency and functionality, which enables tailored performance characteristics in various applications.

The object comprises an implant for medical applications.

The implant comprises a body structure including having first regions 102, preferably porous regions, and second regions 104, preferably solid regions.

The first regions 102 are configured to promote bone ingrowth, and the second regions are configured to provide mechanical stability.

The implant is configured to be used in spinal, foot, ankle, or cranio-maxillofacial (CMF).

In particular, the implant is configured to be used in spinal, foot, ankle, cranio-maxillofacial (CMF), or other orthopedic or medical applications requiring a combination of porosity and mechanical stability.

Advantageously, the mechanical properties of the solid region are optimized for load-bearing and structural support, and the mechanical properties of the porous region are optimized for promoting tissue integration and bone growth.

In particular, the porous regions have a porosity structure that varies across the implant to match specific anatomical requirements.

Advantageously, the implant is designed to enhance biomechanical compatibility with surrounding tissue and bone to ensure long-term stability and integration.

### REFERENCE NUMERALS

- 10, 100: First layer
- 12, 102: First region
- 14, 104: Second region
- 16 106: Transition boundary
- 18: Subsequent layer

- 200: Method
- 202: Method step
- 204: Method step
- 206: Method step

## Claims

1. A method (200) of fabricating an object, such as a three-dimensional object comprising variable-density regions (104), preferably using a fused filament fabrication technique, comprising the steps of:
- depositing (202) a material to create a first layer of the object based on a first pathway in the X, Y and Z dimensions, wherein the first layer (100) comprises first regions (102) including a first infill density, preferably porous regions;
- depositing (204) the material to create second regions (104) by filling selected pores of the porous regions (102) within the first layer (100) based on a second pathway in the X, Y and Z dimensions, wherein the second regions (104) include a second infill density;
wherein the second infill density is greater than the first infill density.

2. The method (200) according to claim 1,
**characterized in that**
wherein the first pathway and the second pathway comprise overlapping segments that cross each other in the X and Y dimensions,
wherein the first pathway and the second pathway further comprise a substantially consistent Z dimension, with a minimal vertical offset.

3. The method (200) according to claim 1 or claim 2,
**characterized in that**
wherein the second regions (104) comprise the second infill density varying between 30% and 40% infill, 40% and 60% infill, or 70% to 100% infill, preferably the second regions (104) comprise solid regions.

4. The method (200) according to any one of preceding claims,
**characterized in that**
the method (200) further comprises repeating (206) the steps of depositing to create a subsequent layer and to fill the selected pores within the first layer (100), and/or
the step of adapting printing parameters for creating the solid regions (104), wherein the printing parameters comprise at least one of extrusion temperature, print speed, and retraction settings.

5. The method (200) according to any one of preceding claims,
**characterized in that**
the material (200) comprises a filament material selected from the group comprising:
- high performance polymers, such as Polyaryletherketones (PAEKs), Polyetheretherketone (PEEK), Polyetherketoneketone (PEKK), Polyphenylsulfone (PPSU), and polymers filled with Biphasic Calcium Phosphate/Hydroxyapatite (BCP/HA),
- bio-degradable polymers, such as poly(L-lactic acid) (PLLA), Poly(D-lactic acid) (PDLA), Poly(lactic-co-glycolic acid) (PLGA), Poly(glycolic acid) (PGA), Poly(dioxanone) (PDO), and Polycaprolactone (PCL),
- polylactic acid (PLA),
- acrylonitrile butadiene styrene (ABS), and
- thermoplastic polyurethane (TPU).

6. The method (200) according to one of the preceding claims,
**characterized in that**
the method (200) further comprises the step of employing a temperature control system to monitor and adjust the nozzle temperature during extrusion.

7. The method (200) to one of the preceding claims,
**characterized in that**
the first layer (100) comprises porous regions (102) including interconnected pores, preferably the first pathway comprises a Gyroid patten, Schwarz pattern, Diamond pattern, a rectilinear refill pattern, a honeycomb pattern, a triangular pattern, a concentric pattern, a zigzag patent or a combination thereof.

8. The method (200) according to one of the preceding claims,
**characterized in that**
the method (200) further comprises the step of preparing, which includes at least one of the following steps:
- providing a thermoplastic filament material;
- feeding the filament material into a print head comprising an extrusion nozzle;
- heating a nozzle of a printer to a temperature sufficient to melt the filament;
- extruding the melted filament through the nozzle onto a build platform;
- utilizing a path tool to define predefined pathways for a print head movement along the X, Y, and Z dimensions; and
- moving the print head along the defined pathways in the X, Y, and Z dimensions to form the object.

9. The method (200) according to claim 8,
**characterized in that**
the method (200) further comprises the steps of:
- utilizing a path tool to define predefined pathways for a printer head movement along the X, Y, and Z dimensions; and
- moving the print head along the defined pathways in the X, Y, and Z dimensions to form the object.

10. The method (200) according to claim 8 or claim 9,
**characterized in that**
the method (200) further comprises the step of cooling the extruded filament to solidify the layer.

11. The method (200) to one of claims 7 to 10,
**characterized in that**
the method (200) further comprises the step of post-processing the printed object to enhance surface finish and mechanical properties.

12. The method (200) according to claim 11,
**characterized in that**
the post-processing comprises at least one of a milling step, a grinding step, and a cutting step.

13. An object fabricated by the method (200) according to one of the preceding claims, wherein the object comprises variable-density regions with seamless transition boundaries (106) between the regions.

14. An object according to claim 13,
**characterized in that**
the object comprises an implant for medical applications,
wherein the implant comprises a body structure including having first regions (102), preferably porous regions, and second regions (104), preferably solid regions, wherein the first regions (102) are configured to promote bone ingrowth, and the second regions are configured to provide mechanical stability.

15. The object of claim 14,
**characterized in that**
the implant is configured to be used in spinal, foot, ankle, or cranio-maxillofacial (CMF).
